**Europäisches Patentamt**

**European Patent Office**

**Office européen des brevets**

(11) Publication number: **0 213 894**
**A2**

# (12) EUROPEAN PATENT APPLICATION

(21) Application number: **86306477.0**

(22) Date of filing: **21.08.86**

(51) Int. Cl.⁴: **A 61 K 39/12**
**A 61 K 39/21**

(30) Priority: **23.08.85 US 768545**

(43) Date of publication of application:
**11.03.87 Bulletin 87/11**

(84) Designated Contracting States:
**AT BE CH DE FR GB IT LI LU NL SE**

(71) Applicant: **ADVANCED GENETICS RESEARCH INSTITUTE**
**Cottonmill Building 2220 Livingston Street**
**Oakland California 94606 (US)**

(72) Inventor: **Wiesehahn, Gary P.**
**1170 9th Street 5**
**Alameda California 94501 (US)**

**Lin, Lily**
**80 Hill Road**
**Berkeley California 94708 (US)**

**Botchan, Michael R.**
**1 Ardmore Path**
**Kensington California 94708 (US)**

(74) Representative: **Harrison, David Christopher et al**
**MEWBURN ELLIS & CO 2/3 Cursitor Street**
**London EC4A 1BQ (GB)**

Claims for the following Contracting State: AT.

(54) **Defective viral particle vaccines and methods for their use.**

(57) Novel viral vaccines comprise defective viral particles in combination with an immunologic adjuvant. In the exemplary embodiment, defective retroviral particles comprise intact retroviral core and envelope proteins and genomic RNA incapable of reproducing the retrovirus. The retroviral particles are produced by co-expression in a suitable host of packaging-deficient retroviral DNA construct(s) and packaging sequence construct(s) which encode the packaged RNA. Defective viral particles may be recovered from the growth media of the expression host.

**Description**

DEFECTIVE VIRAL PARTICLE VACCINES AND METHODS FOR THEIR USE

Viral vaccines require the preparation of an immunogenic substance which is non-pathogenic yet capable of eliciting a protective immune response in an inoculated host. Over the years, a number of approaches have been developed for treating live, infectious viruses to provide the desired non-pathogenic immunogenic substance. For example, viruses have been inactivated by the application of heat, formalin, ozone, and the like, which results in killed viral particles which are incapable of infection. While such killed virus vaccines are generally safe, the immunogenic characteristics of the virus are often degraded, and they are not always effective. Attenuated live viruses, which are living but incapable of causing disease, may also find use as vaccines. While such live virus vaccines will often provide improved immunologic reactivity, the risk of such viruses reverting to a pathogenic form is always present. More recently, subunit vaccines which comprise one or more antigenic proteins or protein fragments characteristic of the virus have found use. Although such vaccines are inherently safe, the stimulation of an immune response against isolated antigens is not always effective in providing complete protection.

For the above reasons, it would be desirable to provide improved viral vaccines which are capable of eliciting an immune response analogous to that of the live virus yet which are non-infectious and virtually incapable of reverting to an infectious state. More particularly, it would be desirable to provide a vaccine which incorporates viral particles comprising intact envelope and coat proteins but which are incapable of mounting a productive infection.

U.S. Patent No. 4,405,712 describes a vector which can activate genes and additional viral sequences comprising the long terminal repeat (LTR) sequence from the Moloney sarcoma virus, which vector can be rescued by helper Moloney leukemia virus. Mann et al. (1983) Cell 33:153-159 describe the construction of a modified Moloney murine leukemia virus with a deletion in the packaging site. Cells carrying the modified virus allow for replication of viruses which carry the packaging site but which are otherwise defective. Watanabe and Temin (1982) Proc. Natl. Acad. Sci. USA 79:5986-5990 define the encapsidation (E) region necessary for packaging of the spleen necrosis virus. Gluzman (1981) Cell 23:175-182 describes the preparation of the COS-1 cell line which contains an integrated copy of the early region of SV40 DNA virus, and which may be used to propagate recombinant SV40 viruses with foreign DNA substituted for the viral early region. Normal viral populations include a high proportion of particles which are defective in that they are unable to mount a productive infection. The remaining portion of the particles, however, are infectious and capable of maintaining and spreading the infection.

Novel compositions and methods are provided for the vaccination of mammalian hosts against viral infection. Compositions consisting of immunogenic, non-infectious viral particles substantially entirely free from infective viral particles are produced through co-expression of two separate DNA constructs. The first construct includes some or all of the information necessary for packaging a viral particle within its antigenic coat or shell, while the second construct provides information for producing substitute viral nucleic acids. The substitute nucleic acids are capable of being packaged within the antigenic coat, but do not encode the coat proteins. Such defective viral particles are thus antigenically intact and immunologically active, while being unable to replicate and mount a productive infection. By further dividing and separating the viral genetic information on each construct, the chance of the viral genes recombining to form a pathogenic viral particle can be reduced to an arbitrarily low value.

In the exemplary embodiment, defective retroviral particles are prepared by expressing packaging-deficient retroviral DNA, including the gag, pol, and env genes, in the presence of non-contiguous DNA packaging sequences. The DNA packaging sequences are copied by the cell into an RNA sequence which does not contain viral genes (e.g. gag, pol or env), but has the viral packaging site. This RNA sequence is then packaged within envelope and core proteins encoded by the packaging-deficient retroviral DNA. This combination of harmless but "packageable" genetic material and a cell producing viral coat proteins results in a system which generates engineered defective particles. These particles look like normal (i.e., pathogenic) virus to the immune system, but because they lack the pathogenic genes inside they are incapable of causing an infection. Vaccines are prepared from the defective retroviral particles and may incorporate an immunologically active adjuvant.

Fig. 1 illustrates the construction of pEVX-neo from MuLV.
Fig. 2 illustrates the construction of pFeVX-neo from FeLV-B.
Fig. 3 illustrates Gardner-Arnstein FeLV, where:
S.D. = Splice Donor.
S.A. = Splice Acceptor.
P.S. = Putative packaging site based on sequence homology with Harvey's MuSV.
H.R. = Homologous region having strong homology with Harvey's MuSV.

DESCRIPTION OF THE SPECIFIC EMBODIMENTS

According to the present invention, defective viral particles are produced which are antigenically intact but lack at least some of the viral genes necessary for replication. Vaccines comprising such defective viral particles and optionally an immunologic adjuvant are capable of eliciting an immune response when administered to a susceptible host, but incapable of mounting a productive infection.

Packaging-deficient viral DNA constructs are produced by separating the viral genes which code for the antigenic core and coat proteins from the packaging sequences necessary to produce an intact viral particle. DNA encoding the packaging sequences is joined to non-homologous DNA to provide a DNA packaging sequence construct which upon transcription furnishes a substitute viral nucleic acid for the defective viral particle. The nucleic acid is of the proper size to be packaged within the viral coat, but is defective in that it lacks at least some and preferably most or all of the genes necessary for replication, particularly lacking the genes coding for the coat and core proteins. Expression of both the packaging-deficient viral DNA and the DNA packaging sequence constructs in the same cell results in the production of particles with the desired viral core and coat proteins packaging an arbitrary nucleotide sequence. Such a "hybrid" virus does not contain the genetic information necessary to replicate itself.

The present invention is useful for producing vaccines to a wide variety of animal viruses, including but not limited to double-stranded DNA viruses, such as adenoviruses, herpesviruses, papovaviruses, and poxviruses; single-stranded RNA viruses, such as picornaviruses, orthomyxoviruses, paramyxoviruses, rhabdoviruses, coronaviruses, arenaviruses, and retroviruses; and double-stranded RNA viruses, such as reoviruses. The viruses may be naked or enveloped. In the case of naked viruses, the packaging deficient DNA construct will code for the capsid and core proteins. For enveloped viruses, the packaging deficient sequence will also code for the envelope proteins.

In the exemplary embodiment hereinafter, a vaccine to feline leukemia virus, a retrovirus, is produced by separating the genes which code for the core proteins, reverse transcriptase and envelope proteins onto the packaging deficient DNA construct and the genes which encode the packaging sequences onto the separate packaging DNA sequence, as will now be described in detail.

Retroviruses are single-stranded RNA viruses which require the synthesis of a DNA intermediate for replication. The RNA genomes, typically comprising from about 8 to 10 kilobases, are packaged within a structure comprising core proteins, reverse transcriptase, and envelope glycoproteins. These proteins are encoded by the genomic RNA. The reverse transcriptase contained in the virus particle provides for initial transcription of linear cDNA which, in turn, serves as a template for production of double-stranded proviral DNA. The proviral DNA is integrated into the genomic DNA of the infected host cell.

During the synthesis and integration of the proviral DNA, long terminal repeats (LTR's) are formed. Each of these LTR's includes sequences involved in transcriptional regulation of mRNA from the integrated genes. In particular, the 5' LTR sequence includes the promoter necessary for transcribing each of the genes, while the 3' LTR includes the polyadenylation sequence necessary for efficient RNA processing. This completed proviral form is subsequently integrated into a host chromosome.

In addition to the above, oncogenic retroviruses contain an oncogene which specifies a protein associated with the neoplastic transformation of an infected host. The oncogene may be inserted into the gag, pol, or env gene. This frequently renders the retroviruses defective so that to mount a productive infection they require a "helper" virus which carries an intact copy of the gene disrupted by insertion of the oncogene. In certain rare instances, the oncogene is a separate (fourth) gene. In at least one case, Rous sarcoma virus, the oncogene is located at the 3'-end of the genomic RNA.

The specific method for producing defective retroviral particles is applicable to all retroviruses which are known to be pathogenic to mammalian hosts. Exemplary among such pathogenic retroviruses are human T cell leukemia virus I (HTLV-I), human T cell leukemia virus III (HTLV-III), feline leukemia virus (FeLV), murine leukemia virus (MuLV), feline sarcoma virus (FeSV), murine sarcoma virus (MuSV), avian leukosis virus (ALV), Rous sarcoma virus (RSV), feline endogenous virus (RD114), hampster leukosis virus (HLV), porcine leukosis virus (PLV), bovine leukosis virus (BLV), equine infectious anemia virus (EIAV), and the like. While the present invention is exemplified by the production of retroviral vaccines, it is also applicable to other viruses as set forth above.

The present invention relies on providing at least two separate DNA constructs which are expressed simultaneously in single host cells. The first DNA construct provides all of the genes required for virus replication and assembly but is too crippled to produce viral particles directly. The second DNA construct is used to isolate bona fide virus particles which are antigenically similar to the live, pathogenic virus on the outside, but which cannot reproduce in the absence of the first construct because they contain the wrong genetic information. For the exemplary case of defective retroviral particles, the first DNA construct, referred to as the packaging-deficient retroviral DNA construct, includes the gag, pol, and env genes of the native proviral form of the retrovirus of interest. The packaging-deficient retroviral DNA construct may be obtained by modifying or altering native proviral DNA in such a way that mRNA transcripts produced from the packaging-deficient retroviral DNA are incapable of being packaged in the viral core and envelope proteins.

Various alterations may be employed. Usually, the packaging site, located near the 5'-end of the genome, is inactivated by mutation or deletion. Such packaging site-deficient (PS-) DNA constructs will provide for production within the host of the necessary packaging proteins, but are incapable of being assembled into viral particles. In addition to inactivating the packaging site, other modifications may be made to the PS-retroviral DNA to assure that it remains packaging-deficient, even if individual alterations revert to the wild type. For example, the 3'-LTR may be removed and replaced with a non-homologous polyadenylation signal. The specific tRNA binding site, located immediately to the 3'-side of the 5'-LTR, may be removed from the PS-retroviral DNA construct to prevent the initiation of replication by RNA-dependent DNA polymerase, and

3

portions of individual genes, such as the p15E region of env, may be deleted. It is preferred to separate the gag, pol, and env genes within the PS− construct or onto separate PS− DNA constructs to assure that the genes are not contiguous. With these modifications, the chance of all three viral genes being recombined onto the same DNA molecule as the package site is extremely remote. All or any combination of such alterations may be employed when preparing the packaging deficient retroviral DNA of the present invention. Usually, alteration of the packaging site together with any one of the other alterations will assure that the retroviral DNA cannot revert to the pathogenic wild-type and be packaged within a viable viral particle.

Portions of the gag, pol and env genes may be derived from different subgroups of FeLV (e.g., FeLV-A, FeLV-B, or FeLV-C subgroups) in order to enhance defective particle production or to enhance antigenicity. Defective particle preparations containing epitopes of different FeLV subgroups may be mixed in order to produce a "multivalent" vaccine to provide protective immunity against more than one FeLV subgroup.

In cases of highly oncogenic retroviruses that carry an oncogene, the oncogene will normally be removed from the proviral DNA in preparing the packaging-deficient retroviral DNA. Since the defective retroviral particles are capable of penetrating the cells of the vaccinated host, the presence of the oncogenic protein is highly undesirable, even though the retroviral particle would be incapable of replication.

The second DNA construct of the present invention is the packaging sequence DNA construct. The packaging sequence DNA construct will encode the RNA transcript which is subsequently packaged within the viral core and envelope proteins encoded by the packaging-deficient retroviral DNA construct. Thus, the packaging sequence DNA construct will include all packaging sequences necessary for packaging, including an intact packaging site recognized by the retroviral system. There is considerable flexibility in the nature of the remaining DNA sequences in the packaging sequence DNA construct. Conveniently, the remaining sequences may include one or more selectable marker(s) which will allow for selection of the packaging sequence construct upon introduction in the expression host and will provide a means to titer vaccine preparations.

Since the packaged RNA may be expressed in some cells in the vaccinated animal, it may be desirable to include sequences which code for at least some of the viral antigens which can elicit serum neutralizing antibodies. For FeLV such a viral antigen may be all or part of the viral coat protein, gp70. More than one viral epitope may be encoded by the packaged RNA, and different epitopes may be encoded by different packaged RNAs. Epitopes from different subgroups of FeLV may be used.

Once the packaging deficient retroviral DNA construct(s) and the packaging sequence construct have been prepared, they are introduced into an expression host by conventional techniques such as transfection or co-transfection with a selectable marker. In the latter case, the two DNA constructs may be introduced simultaneously with a single selective marker, or sequentially employing separate markers for each introduction. In either case, the transfected cells can be screened for the production of defective viral particles as follows: Culture supernatants from clones selected for the marker employed (such as drug resistance) can be monitored for antigen (virus particle) production by ELISA and by reverse transcriptase (RT) activity, another indication of particle concentration. In order to guarantee the absence of any viable recombinants, the clones can be screened for the presence of any live virus by the most sensitive viability assay available for the system, such as (1) a focus-forming assay (for FeLV), (2) back passage on permissive cells followed by ELISA, RT or any suitable assay, or ultimately (3) the ability to cause disease in the appropriate host.

Mammalian cell lines will be employed for expression of the defective viral particles to allow for post-translational modification, e.g., glycosylation, and secretion. A variety of suitable mammalian host cell cultures are available. Conveniently, a mammalian host matching the specificity of the retrovirus will be selected. For example, a feline cell culture will usually be selected for the production of defective FeLV particles. In some cases, however, it might be desirable to employ other host cells for expression of the DNA constructs. When employing such heterologous expression systems, it will often be desirable to provide in the DNA constructs enhancer/promoter sequences which are preferred by the host cells. For example, if FeLV sequences are being expressed in a rat cell culture, it may be desirable to substitute a rat promoter for the FeLV promoter located in the 5'-LTR of the packaging deficient retroviral DNA construct.

Transfected expression hosts are thus capable of producing defective viral particles. The retroviral particles are secreted into the growth medium of the expression host and may be recovered therefrom by conventional techniques.

The defective retroviral particles may be formulated in a variety of ways for use for inoculation. Concentration of the defective viral particles will generally correspond to a live virus concentration of from about $10^6$ to $10^9$ ffu/ml, with a total dosage of at least $10^5$ ffu/dose, usually $10^6$ ffu/dose, preferably at least $10^7$ ffu/dose. The total dosage administered over several injections will usually not exceed about $10^9$ ffu, more usually not exceeding about $10^8$ ffu. The retroviral particles may be combined with a physiologically-acceptable, immunologically active adjuvant, such as mineral oil, vegetable oil, mineral salts, and immunopotentiators, such as muramyl dipeptide. The vaccine may be administered subcutaneously, intramuscularly, intraperitoneally, orally, or nasally. Usually, a specific dosage at a specific site will range from about 0.1 ml to 4 ml, where the total dosage will range from about 0.5 ml to 8 ml. The number of injections and their temporal spacing may be highly variable, but will usually comprise one to three injections at 1, 2, or 3 week intervals.

The following examples are offered by way of illustration, not by way of limitation.

EXPERIMENTAL

Materials and Methods

Bacterial strains

E. coli strain HB101 was used for the growth of plasmids containing the original complete FeLV genome or constructs derived therefrom. For growth of M13 clones, the strain JM103 was employed.

Eukaryotic cells

Transfection experiments were conducted with both feline and rodent cells. The cat cell line routinely employed was CRFK, obtained from the American Type Culture Collection, Rockville, Maryland. It was grown in monolayers on T-25 or T-75 flasks (Corning) in MEM (GIBCO) plus 10% fetal calf serum under 5% $CO_2$ at 37°C. The rat cell line Rat-2 was grown in DME (GIBCO) plus 10% fetal calf serum. Transfections were carried out by the calcium phosphate precipitation method as described by Wigler et al. (1979) Cell 16:777-785.

Plasmids containing a packaging site

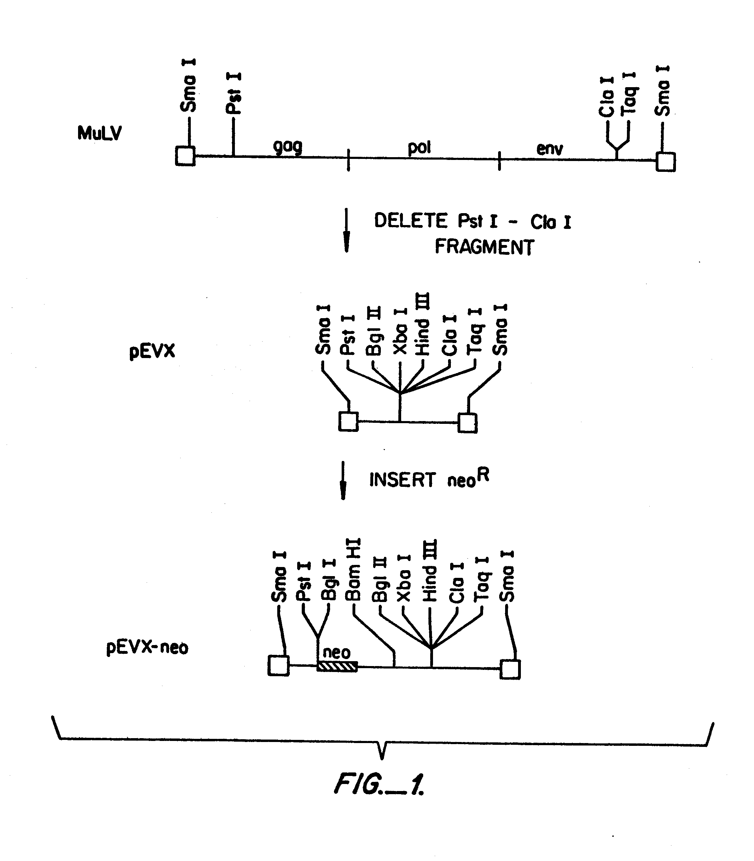

The murine-derived construct pEVX-neo (Kriegler et al. (1984) Cell 38:483-491) was utilized (see Fig. 1). This plasmid was originally constructed by deleting most of the gag, pol and env genes from MuLV and inserting the neomycin-resistance gene of Tn 5 along with a polylinker to facilitate subsequent cloning steps. This plasmid confers resistance to geneticin (G418) upon eukaryotic cells following transfection, and thus allows cells carrying transfected and co-transfected sequences to be selected by growing the cells in 375 μg/ml of G418.

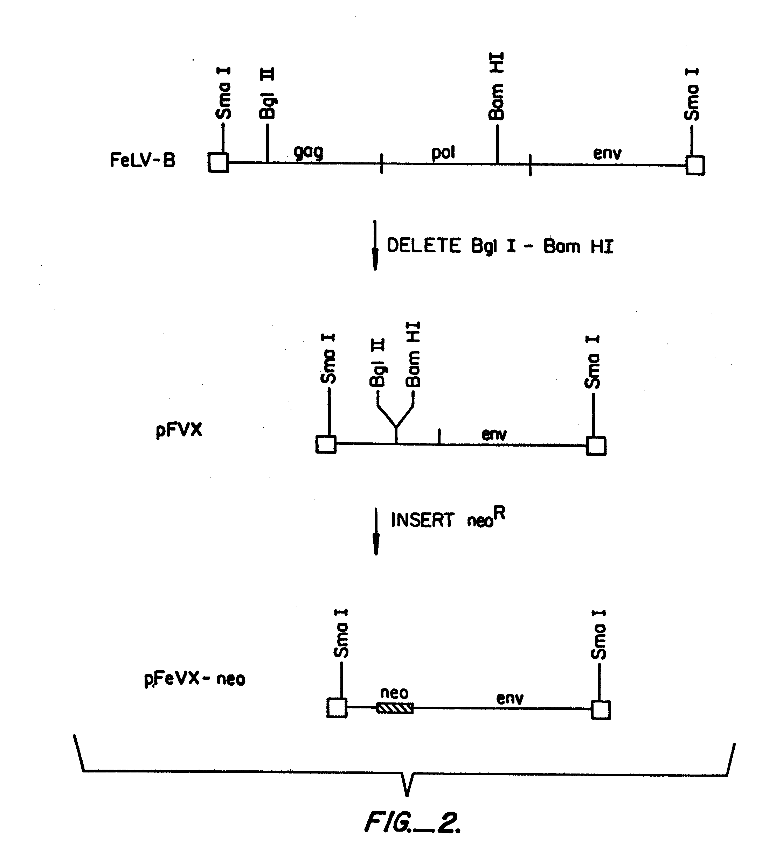

A second vector, termed pFeVX-neo (Fig. 2), was constructed by deleting the gag and pol genes of FeLV-B between the Bgl II and Bam HI restriction sites, and inserting the Bgl II fragment of pEVX-neo containing the Tn 5 neomycin resistance gene.

Construction of packaging deficient FeLV genomes

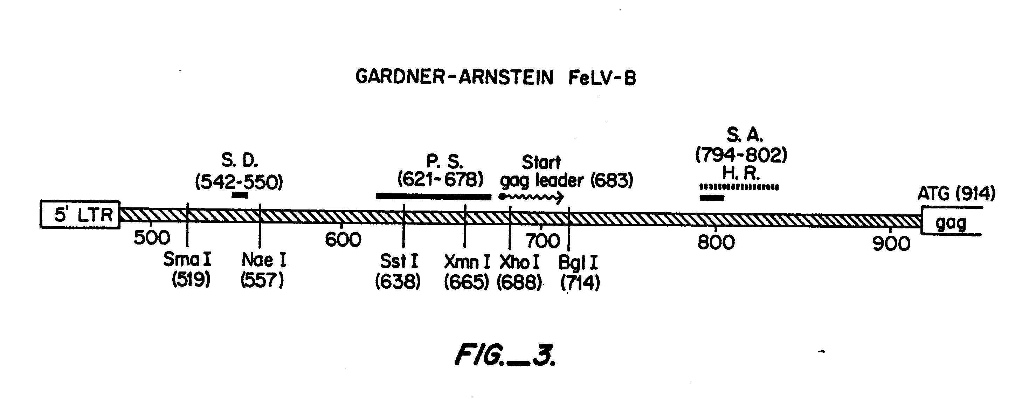

All FeLV-B constructions were derived from a cloned, infectious Gardner-Arnstein feline leukemia virus of subgroup B (Fig. 3). The putative packaging site for FeLV-B was determined based on comparison of sequence homology of FeLV-B and Harvey's murine sarcoma virus. Packaging site deletions were generated for FeLV-B by limited Bal 31 (Maniatis et al., 1983 Molecular Cloning: A Laboratory Manual, Cold Spring Harbor, p.18) digestion from the Bgl I site which is 714 nucleotides from the beginning of U3 and approximately 200 nucleotides 5' to the beginning of the gag gene region (Hampe et al. (1983) J. Virol. 45:466-472). The digestion products were monitored on a gel, used to transform E. coli strain HB101, and isolated as independent bacterial clones resistant to ampicillin. Several of the isolates have been characterized by M13 subcloning and sequencing utilizing standard methods (Messing et al. (1981) Nuc. Acids. Res. 9:309-323, and Sanger et al. (1977) Proc. Natl. Acad. Sci. USA 74:5463-5467) and shown to contain deletions that range in size from 150 to 320 base pairs. Plasmids pNX and pSX were constructed by deleting the fragment between Nae I and Xmn I and the fragment between Sst I and Xmn I, respectively. The restriction endonuclease-generated deletions in the pNX and pSX vectors were also verified using M13 subcloning and sequencing methods (Messing et al., supra.). These two plasmids thus have considerably less information deleted than pDD38. They retain the gag leader and are missing only one of the two regions identified by DNA sequence homology with the murine system. The deletion constructs were as follows:

| Designation | Deletion* |
|---|---|
| DD-15 | 577-803 |
| DD-6 | 619-881 |
| DD-1 | 559-864 |
| DD-19 | 516-910 (approx.) |
| DD-38 | 628-817 |
| LL-1 | 519-869 |
| LL-8 | 519-899 |
| LL-12 | 519-1059 |
| pNX | 557-665 |
| pSX | 634-665 |

\* Numbers indicate the beginning and ending bases of the deletions.

Separation of the gag, pol and env genes onto separate DNA molecules was achieved as follows. Plasmid pBal containing a deletion in the envelope region was constructed from an infectious FeLV-B clone by removing a Bal I fragment containing the env gene. Because sites for many restriction enzymes exist in the FeLV genome, before making many of these constructions, the large Xho I fragment containing most of the FeLV genome was deleted and then reinserted after the desired manipulations were complete. Plasmid pBB, which is missing most of the gag and pol genes, but contains an intact env gene, was constructed by cleaving an infectious FeLV-B clone with Bam HI and Bgl II and subsequent reannealing (Bam HI and Bgl II leave identical overlapping ends).

Detection of FeLV antigens

Defective FeLV particles were detected by ELISA using an FeLV test kit (Leukassay*F, Pitman Moore). The plates are coated with monoclonal antibody to core protein p27, encoded by the gag gene. Additional assays will be developed utilizing purified envelope protein gp70 in order to follow the production of envelope protein independently.

Viability assay for FeLV

Infectious FeLV was monitored by a focus-forming assay on clone 81 cells, a derivative of Crandall's feline kidney (CRFK) cells containing the murine sarcoma virus genome, according to the procedure of Fischinger et al. (1974) J. Virol. 14:177-179. Foci were enumerated on day 12.

RESULTS

Expression of pSX in CRFK cells

CRFK cells were co-transfected with plasmid pSX and either the drug resistance vector pEVX-neo or pFeVX-neo. Clones selected for resistance to G418 were isolated from four T-75 tissue culture flasks and grown to confluency in 60 mm dishes. A total of 22 clones were then analyzed for expression of the FeLV-B genome (ELISA to p27 protein) and for infectious FeLV-B activity (focus forming assay on clone 81 cells). Results are summarized in Table 1.

As shown in Table 1, most of the clones from these transfection experiments are positive by the ELISA assay, indicating the presence of gag-encoded FeLV p27 core protein in the growth medium. It can therefore be concluded that complete viral particles are being produced by these clones, and it appears that the pSX FeLV-B vector is fully capable of expressing the gag, pol, and env gene products.

TABLE 1. Results of screening for pSX expression in
CRFK cells by ELISA and clone 81 focus assay.

| Vector | Flask No. | Clone No. | ELISA | O.D. | Clone 81 Assay (ffu/ml) |
|--------|-----------|-----------|-------|------|-------------------------|
| pSX/pEVX-neo | 1 | VII-5-1 | + | .589 | < 2.5 |
| | | " " 2 | + | .719 | < 2.5 |
| | | " " 3 | + | .771 | < 2.5 |
| | | " " 4 | + | .387 | < 2.5 |
| | | " " 5 | + | .153 | < 2.5 |
| | | " " 6 | + | .424 | < 2.5 |
| | | " " 7 | + | .561 | < 2.5 |
| | | " " 9 | + | .371 | < 2.5 |
| | | " " 10 | + | .400 | < 2.5 |
| pSX/pEVX-neo | 2 | VII-6-1 | − | .078 | < 2.5 |
| | | " " 2 | + | .335 | < 2.5 |
| | | " " 3 | − | .036 | < 2.5 |
| | | " " 4 | + | .336 | $4.5 \times 10^2$ |
| | | " " 5 | − | .080 | < 2.5 |
| pSX/pFeVX-neo | 3 | VII-7-1 | + | .432 | < 2.5 |
| | | " " 2 | + | .937 | < 2.5 |
| | | " " 3 | + | .153 | 20 |
| pSX/pFeVX-neo | 4 | VII-8-1 | − | .101 | $6.5 \times 10^2$ |
| | | " " 2 | + | .556 | < 2.5 |
| | | " " 3 | + | .196 | $6.4 \times 10^2$ |
| | | " " 4 | + | .262 | $1.3 \times 10^3$ |
| | | " " 5 | − | .088 | $1.4 \times 10^2$ |

ELISA and clone 81 data for pSX clones. The O.D. refers to the spectrophotometric optical density reading obtained with supernatants from neomycin-resistant clones subjected to colorimetric p27 ELISA assay.

Positive and negative FeLV-B controls had average O.D. readings of 1.887 and .041, respectively. Wild-type FeLV-B clones consistently read as high as the positive control. O.D. $\geq 0.150$ was arbitrarily scored as " + ". The fact that the ELISA values are lower than positive controls is thought to reflect the lower packaging efficiency of EVX-neo. Clone 81 data are expressed as the number of focus forming units (ffu) per ml of supernatant. The assay is estimated to be able to detect $\geq 2.5$ ffu FeLV/ml. Therefore clones that did not produce foci on clone 81 cells have been scored as having $< 2.5$ ffu/ml. Supernatants from clones resulting from transfection With wild-type FeLV-B characteristically titer between $10^4$ ffu/ml and $10^5$ ffu/ml on the clone 81 assay.

Of the 17 ELISA-positive clones (Table 1), 12 were negative on the clone 81 infectivity assay. Thus, the pSX plasmid gives rise to an FeLV-B virus population which has at least a factor of $10^3$ less infectious activity than wild-type FeLV-B. The combination of high ELISA assay and low clone 81 titer is indicative of the production of defective retroviral particles, and it seems clear that at least some of the sequences necessary for RNA packaging have been deleted in the pSX construction.

Expression of other plasmids in CRFK cells

Plasmids LL-1, LL-8, DD-15, DD-6, DD-19, DD-38, pBB, and pNX were also used to co-transfect CRFK cells with pEVX-neo or pFeVX-neo. Of these, only pBB and pNX were positive by the ELISA assay described above. None of the ELISA-positive cells derived from these transfections were found to have an infectivity below $3 \times 10^3$ ffu/ml as measured by the clone-81 assay. In this respect they were distinct from the pSX constructions that gave rise to negative results in the clone 81 infectivity assay.

Although the foregoing invention has been described in some detail by way of illustration and example for purposes of clarity of understanding, it will be obvious that certain changes and modifications may be practiced within the scope of the appended claims.

## Claims

1. A vaccine for conferring immunity against an animal virus, said vaccine comprising:
defective viral particles having nucleic acids packaged within antigenic coat and core proteins, wherein the coat and core proteins substantially correspond to those of naturally-occurring virus, but wherein the packaged nucleic acids lack at least some of the genes necessary for pathogenic infection.

2. A vaccine as in claim 1, wherein the packaged nucleic acids lack the genes coding for the coat and core proteins.

3. A vaccine as in claim 1, wherein the animal virus is an RNA virus.

4. A vaccine as in claim 3, wherein the RNA virus is a retrovirus.

5. A vaccine as in claim 4, wherein the coat and core proteins include the envelope proteins.

6. A vaccine comprising:
defective retroviral particles produced by co-expression of (1) packaging-deficient retroviral DNA including the gag, pol and env genes, and (2) packaging sequence DNA encoding RNA, wherein the packaged RNA does not code the gag, pol, and env genes .

7. A vaccine as in claim 6, wherein the gag, pol, and env genes are not contiguous and are located on at least two DNA constructs.

8. A vaccine as in claim 6, wherein the packaging-deficient retroviral DNA is produced by alteration of at least one of the packaging sequences, the 3' long terminal repeat (LTR), and the tRNA binding site, in an intact provirus.

9. A vaccine as in claim 8, wherein the 3'-LTR is replaced by a non-homologous polyadenylation signal.

10. A vaccine as in claim 6, wherein the packaging deficient retroviral DNA is integrated into the genome of a mammalian expression host.

11. A vaccine as in claim 6, wherein the packaging sequence DNA includes a selectable marker.

12. A vaccine as in claim 6, wherein the packaging-deficient retroviral DNA encodes the env, pol, and gag genes of feline leukemia virus type A, B, or C, or a combination thereof.

13. A vaccine as in claim 6, wherein the packaged RNA codes for one or more antigenic determinants of FeLV-A, FeLV-B, or FeLV-C which elicits viral neutralizing responses in the host animal.

14. A vaccine as in claim 13, wherein the antigenic determinant is all or part of viral coat protein, gp70.

Claims for the following Contracting State AT

1. A method of making a vaccine for conferring immunity against an animal virus, comprising using defective viral particles having nucleic acids packaged within antigenic coat and core proteins, wherein the coat and core proteins substantially correspond to those of naturally-occurring virus, but wherein the packaged nucleic acids lack at least some of the genes necessary for pathogenic infection.

2. A method as in claim 1, wherein the packaged nucleic acids lack the genes coding for the coat and core proteins.

3. A method as in claim 1, wherein the animal virus is an RNA virus.

4. A method as in claim 3, wherein the RNA virus is a retrovirus.

5. A method as in claim 4, wherein the coat and core proteins include the envelope proteins.

6. A method of making a vaccine comprising using defective retroviral particles produced by co-expression of (1) packaging-deficient retroviral DNA including the gag, pol and env genes, and (2) packaging sequence DNA encoding RNA, wherein the packaged RNA does not code the gag, pol, and env genes.

7. A method as in claim 6, wherein the gag, pol and env genes are not contiguous and are located on at least two DNA constructs.

8. A method as in claim 6, wherein the packaging-deficient retroviral DNA is produced by alteration of at least one of the packaging sequences, the 3' long terminal repeat (LTR), and the tRNA binding site, in an intact provirus.

9. A method as in claim 8, wherein the 3'-LTR is replaced by a non-homologous polyadenylation signal.

10. A method as in claim 6, wherein the packaging deficient retroviral DNA is integrated into the genome of a mammalian expression host.

11. A method as in claim 6, wherein the packaging sequence DNA includes a selectable marker.

12. A method as in claim 6, wherein the packaging-deficient retroviral DNA encodes the env, pol, and gag genes of feline leukemia virus type A, B, or C, or a combination thereof.

13. A method as in claim 6, wherein the packaged RNA codes for one or more antigenic determinants of FeLV-A, FeLV-B, or FeLV-C which elicits viral neutralizing responses in the host signal.

14. A method as in claim 13, wherein the antigenic determinant is all or part of viral coat protein, gp70.

FIG._1.

FIG._2.

GARDNER-ARNSTEIN FeLV-B

S. D. (542-550)  P. S. (621-678)  Start gag leader (683)  S. A. (794-802) H. R.  ATG (914)

5' LTR — 500 — Sma I (519) — Nae I (557) — 600 — Sst I (638) — Xmn I (665) — Xho I (688) — Bgl I (714) — 700 — 800 — 900 — gag

*FIG._3.*